# EUROPEAN PATENT SPECIFICATION

(11) **EP 1 863 530 B1**
(45) Date of publication and mention of the grant of the patent: **14.07.2010**
(21) Application number: 05721046.0
(22) Date of filing: 11.03.2005
(51) Int. Cl.: A61K 39/395, A61P 35/00, A61K 39/00, C07K 16/30

(54) **METHODS FOR DAMAGING CELLS USING EFFECTOR FUNCTIONS OF ANTI-GFRA1 ANTIBODIES**
VERFAHREN ZUR SCHÄDIGUNG VON ZELLEN MIT EFFEKTORFUNKTIONEN VON ANTI-GFRA1-ANTIKÖRPERN
MÉTHODES D'ENDOMAGEMENT DES CELLULES EN UTILISANT LES FONCTIONS EFFECTRICES DES ANTICORPS ANTI-GFRA1

(43) Date of publication of application: 12.12.2007
(73) Proprietor: Oncotherapy Science, Inc., Kawasaki-shi Kanagawa 213-0012 (JP)
(72) Inventor: NAKATSURU, Shuichi, c/o Oncotherapy Science, Inc., Kawasaki-shi, Kanagawa 213-0012 (JP); IWAMOTO, Takashi, c/o Oncotherapy Science, Inc., Kawasaki-shi, Kanagawa 213-0012 (JP); YOSHIKAWA, Megumi, c/o Oncotherapy Science, Inc., Kawasaki-shi, Kanagawa 213-0012 (JP)
(74) Representative: Jaenichen, Hans-Rainer
(86) International application number: PCT/JP2005/004859
(87) International publication number: WO 2006/095446

(56) References cited:
- WO-A-97/33912
- EHRENFELS C W ET AL: "Perturbation of RET signaling in the embryonic kidney." DEVELOPMENTAL GENETICS. 1999, vol. 24, no. 3-4, 1999, pages 263-272, XP008054325 ISSN: 0192-253X
- MATSUO AKINORI ET AL: "Immunohistochemical localization of glial cell line-derived neurotrophic factor family receptor alpha-1 in the rat brain: Confirmation of expression in various neuronal systems" BRAIN RESEARCH, vol. 859, no. 1, 17 March 2000 (2000-03-17), pages 57-71, XP002350428 ISSN: 0006-8993
- WIESENHOFER BETTINA ET AL: "Glial cell line-derived neurotrophic factor (GDNF) and its receptor (GFR-alpha1) are strongly expressed in human gliomas" ACTA NEUROPATHOLOGICA, vol. 99, no. 2, February 2000 (2000-02), pages 131-137, XP002350429 ISSN: 0001-6322
- WATSON, HOPKINS, ROBERTS, STEITZ, WEINER: "Molecular Biology of the Gene" 1988, BENJAMIN/CUMMINGS , MENLO PARK, US , XP002350877 page 842, line 8 - line 16 page 851; tables 23-1
- GESSNER J E ET AL: "The IgG Fc receptor family" ANNALS OF HEMATOLOGY, vol. 76, no. 6, June 1998 (1998-06), pages 231-248, XP002350430 ISSN: 0939-5555

### SEQUENCE LISTING

<110> ONCOTHERAPY SCIENCE, INC.
<120> METHODS FOR DAMAGING CELLS USING EFFECTOR FUNCTIONS OF ANTI-GFRAL ANTIBODIES
<130> ONC-A0501P
<160> 6
<170> PatentIn version 3.1
<210> 1
   <211> 2542
   <212> DNA
   <213> Homo sapiens
<220>
   <221> CDS
   <222> (550).. (1947)
   <223>
<400> 1
   ggccagaaga aatctggcct cggaacacgc cattctccgc gccgcttcca ataaccacta 60
   acatccctaa cgagcatccg agccgagggc tctgctcgga aatcgtcctg gcccaactcg 120
   gcccttcgag ctctcgaaga ttaccgcatc tatttttttt ttcttttttt tcttttccta 180
   gcgcagataa agtgagcccg gaaagggaag gagggggcgg ggacaccatt gccctgaaag 240
   aataaataag taaataaaca aactggctcc tcgccgcagc tggacgcggt cggttgagtc 300
   caggttgggt cggacctgaa cccctaaaag cggaaccgcc tcccgccctc gccatcccgg 360
   agctgagtcg ccggcggcgg tggctgctgc cagacccgga gtttcctctt tcactggatg 420
   gagctgaact ttgggcggcc agagcagcac agctgtccgg ggatcgctgc atgctgagct 480
   ccctcggcaa gacccagcgg cggctcggga tttttttggg ggggcgggga ccagccccgc 540
   gccggcacc atg ttc ctg gcg acc ctg tac ttc gcg ctg ccg ctc ttg gac 591
   Met Phe Leu Ala Thr Leu Tyr Phe Ala Leu Pro Leu Leu Asp
   1 5 10
   ttg ctc ctg tcg gcc gaa gtg agc ggc gga gac cgc ctg gat tgc gtg 639
   Leu Leu Leu Ser Ala Glu Val Ser Gly Gly Asp Arg Leu Asp Cys Val
   15 20 25 30
   aaa gcc agt gat cag tgc ctg aag gag cag agc tgc agc acc aag tac 687
   Lys Ala Ser Asp Gln Cys Leu Lys Glu Gln Ser Cys Ser Thr Lys Tyr
   35 40 45
   cgc acg cta agg cag tgc gtg gcg ggc aag gag acc aac ttc agc ctg 735
   Arg Thr Leu Arg Gln Cys Val Ala Gly Lys Glu Thr Asn Phe Ser Leu
   50 55 60
   gca tcc ggc ctg gag gcc aag gat gag tgc cgc agc gcc atg gag gcc 783
   Ala Ser Gly Leu Glu Ala Lys Asp Glu Cys Arg Ser Ala Met Glu Ala
   65 70 75
   ctg aag cag aag tcg ctc tac aac tgc cgc tgc aag cgg ggt atg aag 831
   Leu Lys Gln Lys Ser Leu Tyr Asn Cys Arg Cys Lys Arg Gly Met Lys
   80 85 90
   aag gag aag aac tgc ctg cgc att tac tgg agc atg tac cag agc ctg 879
   Lys Glu Lys Asn Cys Leu Arg Ile Tyr Trp Ser Met Tyr Gln Ser Leu
   95 100 105 110
   cag gga aat gat ctg ctg gag gat tcc cca tat gaa cca gtt aac agc 927
   Gln Gly Asn Asp Leu Leu Glu Asp Ser Pro Tyr Glu Pro Val Asn Ser
   115 120 125
   aga ttg tca gat ata ttc cgg gtg gtc cca ttc ata tca gat gtt ttt 975
   Arg Leu Ser Asp Ile Phe Arg Val Val Pro Phe Ile Ser Asp Val Phe
   130 135 140
   cag caa gtg gag cac att ccc aaa ggg aac aac tgc ctg gat gca gcg 1023
   Gln Gln Val Glu His Ile Pro Lys Gly Asn Asn Cys Leu Asp Ala Ala
   145 150 155
   aag gcc tgc aac ctc gac gac att tgc aag aag tac agg tcg gcg tac 1071
   Lys Ala Cys Asn Leu Asp Asp Ile Cys Lys Lys Tyr Arg Ser Ala Tyr
   160 165 170
   atc acc ccg tgc acc acc agc gtg tcc aat gat gtc tgc aac cgc cgc 1119
   Ile Thr Pro Cys Thr Thr Ser Val Ser Asn Asp Val Cys Asn Arg Arg
   175 180 185 190
   aag tgc cac aag gcc ctc cgg cag ttc ttt gac aag gtc ccg gcc aag 1167
   Lys Cys His Lys Ala Leu Arg Gln Phe Phe Asp Lys Val Pro Ala Lys
   195 200 205
   cac agc tac gga atg ctc ttc tgc tcc tgc cgg gac atc gcc tgc aca 1215
   His Ser Tyr Gly Met Leu Phe Cys Ser Cys Arg Asp Ile Ala Cys Thr
   210 215 220
   gag cgg agg cga cag acc atc gtg cct gtg tgc tcc tat gaa gag agg 1263
   Glu Arg Arg Arg Gln Thr Ile Val Pro Val Cys Ser Tyr Glu Glu Arg
   225 230 235
   gag aag ccc aac tgt ttg aat ttg cag gac tcc tgc aag acg aat tac 1311
   Glu Lys Pro Asn Cys Leu Asn Leu Gln Asp Ser Cys Lys Thr Asn Tyr
   240 245 250
   atc tgc aga tct cgc ctt gcg gat ttt ttt acc aac tgc cag cca gag 1359
   Ile Cys Arg Ser Arg Leu Ala Asp Phe Phe Thr Asn Cys Gln Pro Glu
   255 260 265 270
   tca agg tct gtc agc agc tgt cta aag gaa aac tac gct gac tgc ctc 1407
   Ser Arg Ser Val Ser Ser Cys Leu Lys Glu Asn Tyr Ala Asp Cys Leu
   275 280 285
   ctc gcc tac tcg ggg ctt att ggc aca gtc atg acc ccc aac tac ata 1455
   Leu Ala Tyr Ser Gly Leu Ile Gly Thr Val Met Thr Pro Asn Tyr Ile
   290 295 300
   gac tcc agt agc ctc agt gtg gcc cca tgg tgt gac tgc agc aac agt 1503
   Asp Ser Ser Ser Leu Ser Val Ala Pro Trp Cys Asp Cys Ser Asn Ser
   305 310 315
   ggg aac gac cta gaa gag tgc ttg aaa ttt ttg aat ttc ttc aag gac 1551
   Gly Asn Asp Leu Glu Glu Cys Leu Lys Phe Leu Asn Phe Phe Lys Asp
   320 325 330
   aat aca tgt ctt aaa aat gca att caa gcc ttt ggc aat ggc tcc gat 1599
   Asn Thr Cys Leu Lys Asn Ala Ile Gln Ala Phe Gly Asn Gly Ser Asp
   335 340 345 350
   gtg acc gtg tgg cag cca gcc ttc cca gta cag acc acc act gcc act 1647
   Val Thr Val Trp Gln Pro Ala Phe Pro Val Gln Thr Thr Thr Ala Thr
   355 360 365
   acc acc act gcc ctc cgg gtt aag aac aag ccc ctg ggg cca gca ggg 1695
   Thr Thr Thr Ala Leu Arg Val Lys Asn Lys Pro Leu Gly Pro Ala Gly
   370 375 380
   tct gag aat gaa att ccc act cat gtt ttg cca ccg tgt gca aat tta 1743
   Ser Glu Asn Glu Ile Pro Thr His Val Leu Pro Pro Cys Ala Asn Leu
   385 390 395
   cag gca cag aag ctg aaa tcc aat gtg tcg ggc aat aca cac ctc tgt 1791
   Gln Ala Gln Lys Leu Lys Ser Asn Val Ser Gly Asn Thr His Leu Cys
   400 405 410
   att tcc aat ggt aat tat gaa aaa gaa ggt ctc ggt gct tcc agc cac 1839
   Ile Ser Asn Gly Asn Tyr Glu Lys Glu Gly Leu Gly Ala Ser Ser His
   415 420 425 430
   ata acc aca aaa tca atg gct gct cct cca agc tgt ggt ctg agc cca 1887
   Ile Thr Thr Lys Ser Met Ala Ala Pro Pro Ser Cys Gly Leu Ser Pro
   435 440 445
   ctg ctg gtc ctg gtg gta acc gct ctg tcc acc cta tta tct tta aca 1935
   Leu Leu Val Leu Val Val Thr Ala Leu Ser Thr Leu Leu Ser Leu Thr
   450 455 460
   gaa aca tca tag ctgcattaaa aaaatacaat atggacatgt aaaaagacaa 1987
   Glu Thr Ser
   465
   aaaccaagtt atctgtttcc tgttctcttg tatagctgaa attccagttt aggagctcag 2047
   ttgagaaaca gttccattca actggaacat tttttttttt tccttttaag aaagcttctt 2107
   gtgatccttc ggggcttctg tgaaaaacct gatgcagtgc tccatccaaa ctcagaaggc 2167
   tttgggatat gctgtatttt aaagggacag tttgtaactt gggctgtaaa gcaaactggg 2227
   gctgtgtttt cgatgatgat gatgatcatg atgatgatga ttttaacagt tttacttctg 2287
   gcctttccta gctagagaag gagttaatat ttctaaggta actcccatat ctcctttaat 2347
   gacattgatt tctaatgata taaatttcag cctacattga tgccaagctt ttttgccaca 2407
   aagaagattc ttaccaagag tgggctttgt ggaaacagct ggtactgatg ttcaccttta 2467
   tatatgtact agcattttcc acgctgatgt ttatgtactg taaacagttc tgcactcttg 2527
   tacaaaagaa aaaac 2542
<210> 2
   <211> 465
   <212> PRT
   <213> Homo sapiens
<400> 2
   Met Phe Leu Ala Thr Leu Tyr Phe Ala Leu Pro Leu Leu Asp Leu Leu
   1 5 10 15
   Leu Ser Ala Glu Val Ser Gly Gly Asp Arg Leu Asp Cys Val Lys Ala
   20 25 30
   Ser Asp Gln Cys Leu Lys Glu Gln Ser Cys Ser Thr Lys Tyr Arg Thr
   35 40 45
   Leu Arg Gln Cys Val Ala Gly Lys Glu Thr Asn Phe Ser Leu Ala Ser
   50 55 60
   Gly Leu Glu Ala Lys Asp Glu Cys Arg Ser Ala Met Glu Ala Leu Lys
   65 70 75 80
   Gln Lys Ser Leu Tyr Asn Cys Arg Cys Lys Arg Gly Met Lys Lys Glu
   85 90 95
   Lys Asn Cys Leu Arg Ile Tyr Trp Ser Met Tyr Gln Ser Leu Gln Gly
   100 105 110
   Asn Asp Leu Leu Glu Asp Ser Pro Tyr Glu Pro Val Asn Ser Arg Leu
   115 120 125
   Ser Asp Ile Phe Arg Val Val Pro Phe Ile Ser Asp Val Phe Gln Gln
   130 135 140
   Val Glu His Ile Pro Lys Gly Asn Asn Cys Leu Asp Ala Ala Lys Ala
   145 150 155 160
   Cys Asn Leu Asp Asp Ile Cys Lys Lys Tyr Arg Ser Ala Tyr Ile Thr
   165 170 175
   Pro Cys Thr Thr Ser Val Ser Asn Asp Val Cys Asn Arg Arg Lys Cys
   180 185 190
   His Lys Ala Leu Arg Gln Phe Phe Asp Lys Val Pro Ala Lys His Ser
   195 200 205
   Tyr Gly Met Leu Phe Cys Ser Cys Arg Asp Ile Ala Cys Thr Glu Arg
   210 215 220
   Arg Arg Gln Thr Ile Val Pro Val Cys Ser Tyr Glu Glu Arg Glu Lys
   225 230 235 240
   Pro Asn Cys Leu Asn Leu Gln Asp Ser Cys Lys Thr Asn Tyr Ile Cys
   245 250 255
   Arg Ser Arg Leu Ala Asp Phe Phe Thr Asn Cys Gln Pro Glu Ser Arg
   260 265 270
   Ser Val Ser Ser Cys Leu Lys Glu Asn Tyr Ala Asp Cys Leu Leu Ala
   275 280 285
   Tyr Ser Gly Leu Ile Gly Thr Val Met Thr Pro Asn Tyr Ile Asp Ser
   290 295 300
   Ser Ser Leu Ser Val Ala Pro Trp Cys Asp Cys Ser Asn Ser Gly Asn
   305 310 315 320
   Asp Leu Glu Glu Cys Leu Lys Phe Leu Asn Phe Phe Lys Asp Asn Thr
   325 330 335
   Cys Leu Lys Asn Ala Ile Gln Ala Phe Gly Asn Gly Ser Asp Val Thr
   340 345 350
   Val Trp Gln Pro Ala Phe Pro Val Gln Thr Thr Thr Ala Thr Thr Thr
   355 360 365
   Thr Ala Leu Arg Val Lys Asn Lys Pro Leu Gly Pro Ala Gly Ser Glu
   370 375 380
   Asn Glu Ile Pro Thr His Val Leu Pro Pro Cys Ala Asn Leu Gln Ala
   385 390 395 400
   Gln Lys Leu Lys Ser Asn Val Ser Gly Asn Thr His Leu Cys Ile Ser
   405 410 415
   Asn Gly Asn Tyr Glu Lys Glu Gly Leu Gly Ala Ser Ser His Ile Thr
   420 425 430
   Thr Lys Ser Met Ala Ala Pro Pro Ser Cys Gly Leu Ser Pro Leu Leu
   435 440 445
   Val Leu Val Val Thr Ala Leu Ser Thr Leu Leu Ser Leu Thr Glu Thr
   450 455 460
   Ser
   465
<210> 3
   <211> 20
   <212> DNA
   <213> Artificial
<220>
   <223> An artificially synthesized primer sequence for RT-PCR.
<400> 3
   ctgaagcaga agtcgctcta 20
<210> 4
   <211> 20
   <212> DNA
   <213> Artificial
<220>
   <223> An artificially synthesized primer sequence for RT-PCR.
<400> 4
   gacagctgct gacagacctt 20
<210> 5
   <211> 20
   <212> DNA
   <213> Artificial
<220>
   <223> An artificially synthesized primer sequence for RT-PCR.
<400> 5
   gtcagtggtg gacctgacct 20
<210> 6
   <211> 23
   <212> DNA
   <213> Artificial
<220>
   <223> An artificially synthesized primer sequence for RT-PCR.
<400> 6
   ggttgagcac agggtacttt att 23

## Description

### Technical Field

The present invention relates to methods for damaging cells using the effector function of anti-GFRA1 antibodies, or to compositions for this purpose.

### Background Art

Breast cancer, a genetically heterogeneous disease, is the most common malignancy in women. An estimation of approximately 800000 new cases was reported each year worldwide (Parkin DM, Pisani P, Ferlay J (1999). CA Cancer J Clin 49: 33-64). Mastectomy is the first concurrent option for the treatment of this disease. Despite surgical removal of the primary tumors, relapse at local or distant sites may occur due to undetectable micrometastasis (Saphner T, Tommey DC, Gray R (1996). J Clin Oncol, 14,2738-2749.) at the time of diagnosis. Cytotoxic agents are usually administered as adjuvant therapy after surgery aiming to kill those residual or premalignant cells.

Treatment with conventional chemotherapeutic agents is often empirical and is mostly based on histological tumor parameters, and in the absence of specific mechanistic understanding. Target-directed drugs are therefore becoming the bedrock treatment for breast cancer. Tamoxifen and aromatase inhibitors, two representatives of its kind, have been proved to have great responses used as adjuvant or chemoprevention in patients with metastasized breast cancer(Fisher B, Costantino JP, Wickerham DL, Redmond CK, Kavanah M, Cronin WM, Vogel V, Robidoux A, Dimitrov N, Atkins J, Daly M, Wieand S, Tan-Chiu E, Ford L, Wolmark N (1998). J Natl Cancer Inst, 90,1371-1388 ; Cuzick J (2002). Lancet 360, 817-824). However the drawback is that only patients expressed estrogen receptors are sensitive to these drugs. A recent concerns were even raised regarding their side effects particularly lay on the possibility of causing endometrial cancer for long term tamoxifen treatment as well as deleterious effect of bone fracture in the postmenopausal women in aromatase prescribed patients(Coleman RE (2004). Oncology. 18 (5 Suppl 3),16-20). Owing to the emergence of side effect and drug resistance, it is obviously necessarily to search novel molecular targets for selective smart drugs on the basis of characterized mechanisms of action.

Gastric cancer is a leading cause of cancer death in the world, particularly in the Far East, with approximately 700,000 new cases diagnosed worldwide annually. Surgery is the mainstay in terms of treatment, because chemotherapy remains unsatisfactory. Gastric cancers at an early stage can be cured by surgical resection, but prognosis of advanced gastric cancers remains very poor.

Hepatocellular carcinoma (HCC) is one of the most common cancers worldwide and its incidence is gradually increasing in Japan as well as in United States (Akriviadis EA, et al., Br J Surg. 1998 Oct;85(10):1319-31). Although recent medical advances have made great progress in diagnosis, a large number of patients with HCCs are still diagnosed at advanced stages and their complete cures from the disease remain difficult. In addition, since patients with hepatic cirrhosis or chronic hepatitis have a high risk to HCCs, they may develop multiple liver tumors, or new tumors even after complete removal of initial tumors. Therefore development of highly effective chemotherapeutic drugs and preventive strategies are matters of pressing concern.

Renal cell carcinoma (RCC) is the third most common malignancy of the genitourinary system and corresponds to 2-3% of all human malignancies. Surgical resection is the most effective treatment for patients with localized RCC tumors, but such treatment for patients with advanced-stage RCC is not satisfactory. Although some biomedical therapies have been reported to show ∼20% response rate, they often cause severe adverse reactions and do not generally improve patients' survival. Among patients who have surgical treatment, approximately 25-30% relapse after surgery (Ljungberg B., Alamdari F. I., Rasmuson T. & Roos G. Follow-up guidelines for nonmetastatic renal cell carcinoma based on the occurrence of metastases after radical nephrectomy. BJU Int. 84, 405-411 (1999); Levy DA., Slaton JW., Swanson DA. & Dinney CP. Stage specific guidelines for surveillance after radical nephrectomy for local renal cell carcinoma. J Urol. 159, 1163-1167 (1998)). Tumor stage and surgical respectability are the most important prognostic factors for RCC; however, to date, little is known of the underlying molecular mechanisms that influence this variety in prognoses.

RCC tumors can be subdivided on the basis of histological features into clear cell (80%), papillary (∼10%), chromophobe (< 5%), granular, spindle and cyst-associated carcinomas (5-15%). Each of these histological subtypes shows unique clinical behavior, with clear-cell and granular types tending to show more aggressive clinical phenotypes.

Lung cancer is one of the most common lethal human tumors. Non-small-cell lung cancer (NSCLC) is the most common form, accounting for nearly 80% of lung tumors (American Cancer Society, Cancer Facts and Figures 2001, Am. Chem. Soc. Atlanta, 2001). The majority of NSCLCs are not diagnosed until an advanced stage, and thus the overall 10-year survival rate has stayed low at 10%, despite recent advances in multimodality therapies (Fry et al., Cancer, 86: 1867-76, 1999). Currently, chemotherapy using platinum is considered to be a fundamental therapy for NSCLCs. However, the therapeutic action of pharmaceutical agents has not progressed beyond the point of being able to prolong the survival of advanced NSCLC patients to a certain extent (Chemotherapy in non-small cell lung cancer: a meta-analysis using updated data on individual patients from 52 randomized clinical trials, Non-small Cell Lung Cancer Collaborative Group, Bmj. 311: 899-909, 1995). A number of targeting therapies are being investigated, including those that use tyrosine kinase inhibitors. However, to date, promising results have been achieved only in a limited number of patients, and in some patients, therapeutic effects have accompanied severe side effects (Kris et al., Proc Am Soc Clin Oncol, 21: 292a(A1166), 2002).

Research aiming at the elucidation of carcinogenic mechanisms has revealed a number of candidate target molecules for anti-tumor agents. For example, the farnesyltransferase inhibitor (FTI) is effective in the therapy of Ras-dependent tumors in animal models (Sun J, et al., Oncogene. 1998 Mar;16(11):1467-73). This pharmaceutical agent was developed to inhibit growth signal pathways related to Ras, which is dependant on post-transcriptional farnesylation. Human clinical trials where anti-tumor agents were applied in combination with the anti-HER2 monoclonal antibody trastuzumab with the aim of antagonizing the proto-oncogene HER2/neu have succeeded in improving clinical response, and improved the overall survival rate of breast cancer patients (Molina MA, et al., Cancer Res. 2001 Jun 15; 61(12) :4744-9).

Tyrosine kinase inhibitor STI-571 is an inhibitor which selectively deactivates bcr-ab1 fusion protein. This pharmaceutical agent was developed for the therapy of chronic myeloid leukemia, where the constant activation of bcr-abl tyrosine kinase has a significant role in the transformation of white blood cells. Such pharmaceutical agents are designed to inhibit the carcinogenic activity of specific gene products (O'Dwyer ME & Druker BJ. Curr Opin Oncol. 2000 Nov; 12(6):594-7). Thus, in cancer cells, gene products with promoted expression are usually potential targets for the development of novel anti-tumor agents.

Another strategy for cancer therapy is the use of antibodies which bind to cancer cells. The following are representative mechanisms of antibody-mediated cancer therapy:

Missile therapy: in this approach a pharmaceutical agent is bound to an antibody that binds specifically to cancer cells, and the agent then acts specifically on the cancer cells. Even agents with strong side effects can be made to act intensively on the cancer cells. In addition to pharmaceutical agents, there are also reports of approaches where precursors of pharmaceutical agents, enzymes which metabolize the precursors to an active form, and so on are bound to the antibodies.

The use of antibodies which target functional molecules: this approach inhibits the binding between growth factors and cancer cells using, for example, antibodies that bind growth factor receptors or growth factors. Some cancer cells proliferate depending on growth factors. For example, cancers dependent on epithelial growth factor (EGF) or vascular endothelial growth factor (VEGF) are known. For such cancers, inhibiting the binding between a growth factor and cancer cells can be expected to have a therapeutic effect.

Antibody cytotoxicity: antibodies that bind to some kinds of antigens can comprise cytotoxicity to cancer cells. With these types of antibodies, the antibody molecule itself comprises a direct anti-tumor effect. Antibodies that display cytotoxicity to cancer cells are gaining attention as antibody agents expected to be highly effective against tumors. Glial cell line-derived neurotrophic factor family receptor alpha 1 (GFRA1) is found to be expressed in the developing kidney (EHRENFELS, et al., Dev Genet. 1999; 24(3-4):263-72. A polyclonal anti-GFRA1 antibody is provided (EHRENFELS, et al.; MATSUO, et al., Brain Res. 2000 Mar 17; 859(1):57-71; KLEIN RD, et al., WO 97/33912; and WIESENHOFER, et al., Acta Neuropathol. 2000 Feb; 99(2):131-7). KLEIN RD teaches the therapeutic application of GFRA1 neutralizing antibodies in the treatment of the cell tumors. However, in KLEIN RD, there is no Example to indicate the therapeutic effect of GFRA1 neutralizing antibodies.

### Disclosure of the Invention

The present inventors investigated antibodies able to induce cytotoxity, targeting genes showing increased expression in cells. The results revealed that potent cytotoxicity can be induced in GFRA1-expressing cells when those cells are contacted with anti-GFRA1 antibodies, thus completing the present invention.

Specifically, the present invention relates to the following pharmaceutical compositions or methods:
[1] Pharmaceutical compositions comprising an anti-GFRA1 antibody as an active ingredient, wherein the anti-GFRA1 antibody damages (i.e., kills the cell, is toxic to the cell, or otherwise inhibits growth or cell division), an GFRA1-expressing cell using the antibody effector function.
[2] The pharmaceutical compositions are used to treat any pathological condition associated with GFRA1-expressing cells. In typical embodiments, the cell is a cancer cell, such as breast, gastric, liver, renal or lung cancer cell.
[3] The antibodies in the pharmaceutical compositions of the invention are typically monoclonal antibodies.
[4] In some embodiments, the antibody of the invention comprises an effector function such as antibody-dependent cytotoxicity, complement-dependent cytotoxiciy, or both.
[5] Methods for damaging an GFRA1-expressing cell, will comprise the steps of:
   a) contacting the GFRA1-expressing cell with an auti-GFRA1 antibody. As a result of the binding of the antibody the effector function of the antibody will cause damage (i.e., cytotoxicity) to the GFRA1-expressing cell.
[6] Immunogenic compositions for inducing an antibody that comprises an effector function against an GFRA1-expressing cell. The compositions typically comprise as an active ingredient, a GFRA1 polypeptide, an immunologically active fragment thereof, or a nucleic acid molecule the expresses the polypeptides or fragments.
[7] Methods for inducing an antibody that comprises an effector function against an GFRA1-expressing cell, wherein the method comprises administering a GFRA1 polypeptide, an immunologically active fragment thereof, or a cell or a DNA that can express the polypeptides or fragments.

The present invention relates to pharmaceutical compositions for damaging GFRA1-expressing cells using antibody effector function, wherein the compositions comprise as an active ingredient an anti-GFRA1 antibody. The present invention also relates to uses of an anti-GFRA1 antibody to produce pharmaceutical compositions for damaging GFRA1-expressing cells using the anti-GFRA1 antibody effector function. The pharmaceutical compositions of the present invention comprise anti-GFRA1 antibodies and pharmaceutically acceptable carriers. The present inventors used cDNA microarrays for gene expression analysis of breast cancer cells and normal cells collected from breast cancer patients.

A number of genes with specifically enhanced expression in breast cancer cells were subsequently identified. Of these genes with altered expression in breast cancer cells, one gene, glial cell line-derived neurotrophic factor (GDNF) family receptor alpha 1 (GFRA1) gene encoding cytoplasmic membrane protein with low levels of expression in major organs was selected as a candidate target gene for cancer therapies. By selecting genes with low levels of expression in major organs, it was thought that the danger of side effects could be avoided. Among the protein encoded by the genes selected in this way, anti-GFRA1 antibodies were confirmed to have effector functions against GFRA1-expressing cells. In addition, a similar effect was confirmed in other cancer cell lines, such as the gastric, liver, renal, and lung cancer cell lines that this gene over-expressed.

The findings obtained by the present inventors show that, in a forced expression system, GFRA1 tagged with c-myc-His was localized in cytoplasmic membrane, which was confirmed using Immuno-fluorescence microscopy. The GFRA1 gene encodes an amino acid sequence expected to comprise a signal peptide at its N-terminal. As mentioned above, this protein was observed to be chiefly localized in the cytoplasmic membrane, and thus it was thought to be a transmembrane protein. In addition, the low expression level of this gene in major organs, and its high expression in breast cancer cells, establishes that GFRA1 is useful as a clinical marker and therapeutic target.

Conditions required for destroying cancer cells using effector function are, for example, the following:
- Expression of large numbers of antigenic molecules on the membrane surface of cancer cells,
- Uniform distribution of antigens within cancerous tissues,
- Lingering of antigens bound to antibodies on the cell surface for a long time.

More specifically, for example, antigens recognized by antibodies must be expressed on the surface of the cell membrane. In addition, it is preferable that the ratio of antigen-positive cells is as high as possible in cells forming cancerous tissues. In an ideal situation, all cancer cells are antigen-positive. When antigen-positive and negative cells are mixed in cancer cell populations, the clinical therapeutic effect of the antibodies may not be expected.

Usually, when as many molecules as possible are expressed on the cell surface, potent effector functions can be expected. It is also important that antibodies bound to antigens are not taken up into cells. Some receptors are taken up into cells (endocytosis) after binding to a ligand. Equally, antibodies bound to cell surface antigens can also be taken up into the cell. This kind of phenomenon, whereby antibodies are taken up into cells, is called internalization. When internalization occurs, the antibody constant (Fc) region is taken up into the cell. However, cells or molecules essential to effector function are outside the antigen-expressing cells. Thus, internalization inhibits antibody effector function. Therefore, when expecting antibody effector function, it is important to select an antigen that causes less antibody internalization. The present inventors revealed for the first time that GFRA1 is a target antigen possessing such a property.

"Effector function" in the present invention refers to cytotoxicity involved with the Fc regions of antibodies. Alternatively, functions that drive the effect whereby the Fc regions of antibodies bound to antigens damage cells comprising those antigens, can also be referred to as antibody effector function. Specifically, Antibody Dependent Cell-mediated Cytotoxicity (ADCC), Complement Dependent Cytotoxicity (CDC), and neutralizing activity are known as antibody effector functions. Each function is described below.

Antibody Dependent Cell-mediated Cytotoxicity (ADCC):
Cells exist which comprise Fc receptors specific to the Fc region of immunoglobulin classes IgG, IgE, or IgA. Cells that comprise a corresponding Fc receptor recognize and bind to antibodies bound to cell membranes or so on. For example, an IgG class antibody is recognized by Fc receptors on T cells, NK cells, neutrophils, and macrophages. These cells bind to and are activated by the Fc region of IgG class antibodies, and express cytotoxicity against cells to which these antibodies have bound. Cells which acquire cytotoxicity via antibody effector function are called effector cells. ADCC may be divided based on the type of effector cell, as follows:
   ADMC: IgG-dependent macrophage-mediated cytotoxicity, and
   ADCC: IgG-dependent NK-cell-mediated cytotoxicity.

There is no limitation on types of effector cells in the ADCC of the present invention. In other words, the ADCC of the present invention also comprises ADMC, where macrophages are the effector cells.

Antibody ADCC is known to be an important mechanism of the anti-tumor effects, particularly in cancer therapies that use antibodies (Nature Med., 6: 443-446, 2000). For example, a close relationship between the therapeutic effect of anti-CD20 antibody chimeric antibodies and ADCC has been reported (Blood, 99: 754-758, 2002). Thus ADCC is also particularly important among antibody effector functions in the present invention.

For example, ADCC is thought to be an important mechanism in the anti-tumor effects of Rituxan, Herceptin, and so on, for which clinical application has already begun. Rituxan and Herceptin are therapeutic agents for non-Hodgkin's lymphoma and metastatic breast cancer, respectively.

At present, the mechanism for ADCC-mediated cytotoxicity is roughly explained as follows: effector cells, which are bridged to target cells via antibodies bound to the cell surface, are thought to induce target cell apoptosis by transmitting some sort of lethal signal to the target cells. In any case, antibodies that induce cytotoxicity by effector cells are comprised in the antibodies that comprise effector function of the present invention.

### Complement dependent cytotoxicity (CDC):

The Fc regions of immunoglobulins bound to antigens are known to activate complementary pathways. It has also been revealed that the activation pathway may differ depending on the class of immunoglobulin. For example, of the human antibodies, IgM and IgG activate the classical pathway. On the other hand, IgA, IgD, and IgE do not activate this pathway. The activated complements produce, via a number of reactions, a C5b-9 membrane attack complex (MAC) comprising cell membrane-damaging activity. MACs generated in this way are thought to damage viral particles and cell membranes, independently of effector cells. The mechanism for MAC-mediated cytotoxicity is based on the following. MACs comprise a strong binding affinity for cell membranes. MACs bound to a cell membrane open a hole in the cell membrane, making it easy for water to flow in and out of the cell. As a result, the cell membrane is destabilized, or the osmotic pressure is changed, and the cell is destroyed. Cytotoxicity due to an activated complement only extends to membrane close to the antibody which has bound the antigen. For this reason, MAC-mediated cytotoxicity is dependent on antibody specificity. ADCC and CDC can express cytotoxicity independent of each other. However, in practice, these cytotoxicities may function in composite in living bodies. Neutralizing activity:

Antibodies exist which have the function of depriving infectivity of pathogens and activity of toxins. Antibody-mediated neutralization can be achieved by binding of an antigenic variable region to an antigen, or can require complement mediation. For example, in some cases, anti-viral antibodies require complement mediation in order to deprive a virus of its infectivity. Fc regions are essential to the participation of complements. Thus, such antibodies comprise effector function that requires Fc for neutralizing viruses and cells.

In the present invention, effector function can also be explained as a role that determines the biological activity triggered by antigen recognition of an antibody. Herein, preferable target cells are cancer cells. In addition, effector cell functions carried out by the Fc regions of various antibodies rely heavily on antibody class. The Fc region of IgG, IgE, and IgA class antibodies each binds to a specific Fc receptor, and, for example, activates cells that have Fc receptors, and functions in intercellular antibody transport. In particular, IgG class antibodies activate effector cells via Fc receptors on these cells, and then kill target cells to which the variable regions of the antibodies are bound. This is called antibody-dependent cell-mediated cytotoxicity (ADCC). In ADCC, T cells, NK cells, neutrophils, macrophages, or such function as effector cells. On the other hand, the function of activating complement is limited to IgM and IgG class antibodies. Particularly, the function of lysing cells to which antibody variable regions are bound is called complement-dependent cytotoxicity (CDC).

Of these, preferable effector functions herein are either ADCC or CDC, or both. The present invention is based on the fmding that anti-GFRA1 antibodies bind to GFRA1-expressing cells, and then express effector function.

The present invention also relates to methods for damaging GFRA1-expressing cells, which comprise the following steps:
1) contacting the GFRA1-expressing cells with anti-GFRA1 antibodies, and
2) using the effector function of the antibodies which have bound to the GFRA1-expressing cells to damage the cells.

In the methods or pharmaceutical compositions of the present invention, any GFRA1-expressing cell can be damaged or killed. For example, breast, gastric, liver, renal or lung cancer cells are preferable as the GFRA1-expressing cells of the present invention. Of these, breast adenocarcinoma, breast carcinoma, adenosquamous carcinoma of the stomach, hepatocellular carcinoma (HCC), renal cell adenocarcinoma (RCC), or non-small cell lung cancer (NSCLC) cells are preferable.

Cells and antibodies can be contacted *in vivo* or *in vitro.* When targeting *in vivo* cancer cells as the GFRA1-expressing cells, the methods of the present invention are in fact therapeutic methods or preventative methods for cancers. Specifically, the present invention provides therapeutic methods for cancers which comprise the following steps:
1) administering an antibody that binds GFRA1 to a cancer patient, and
2) damaging cancer cells using the effector function of the antibody bound to those cells.

The present inventors confirmed that antibodies binding GFRA1 effectively damage GFRA1-expressing cells, in particular, breast, gastric, liver, renal or lung cancer cells using effector function. The present inventors also confirmed that GFRA1 is highly expressed in breast, gastric, liver, renal or lung cancer cells, with a high probability. In addition, GFRA1 expression levels in normal tissues are low. Putting this information together, methods of breast, gastric, liver, renal or lung cancer therapy where anti-GFRA1 antibody is administered can be effective, with little danger of side effects.

The antibodies of the present invention are not limited so long as they comprise a desired effector function. For example, antibodies comprising the Fc region of IgA, IgE, or IgG are essential for expressing ADCC. Equally, the antibody Fc region of IgM or IgG is preferable for expressing CDC. Therefore, human-derived antibodies belonging to these classes are preferable in the present invention. Human antibodies can be acquired using antibody-producing cells harvested from humans, or chimeric animals transplanted with human antibody genes (Cloning and Stem Cells., 4: 85-95, 2002).

Furthermore, antibody Fc regions can link with arbitrary variable regions. Specifically, chimeric antibodies wherein the variable regions of different animal species are bound to human constant regions are known. Alternatively, a human-human chimeric antibody can also be acquired by binding human-derived variable regions to arbitrary constant regions. In addition, CDR graft technology, where complementarity determining regions (CDRs) composing human antibody variable regions are replaced with CDRs of heterologous antibodies, is also known ("Immunoglobulin genes", Academic Press (London), pp260-274, 1989; Proc. Natl. Acad. Sci. USA., 91: 969-973, 1994). By replacing CDRs, antibody binding specificity is replaced. That is, human GFRA1 will be recognized by humanized antibodies in which the CDR of human GFRA1-binding antibodies has been transferred. The transferred antibodies can also be called humanized antibodies. Antibodies thus-obtained and equipped with an Fc region essential to effector function can be used as the antibodies of the present invention, regardless of the origin of their variable regions. For example, antibodies comprising a human IgG Fc are preferable in the present invention, even if their variable regions comprise an amino acid sequence derived from an immunoglobulin of another class or another species.

The antibodies of the present invention may be monoclonal antibodies or polyclonal antibodies. Even when administering to humans, human polyclonal antibodies can be derived using the above-mentioned animals transferred with a human antibody gene. Alternatively, immunoglobulins which have been constructed using genetic engineering techniques, such as humanized antibodies, human-non-human chimeric antibodies, and human-human chimeric antibodies, can be used. Furthermore, methods for obtaining human monoclonal antibodies by cloning human antibody-producing cells are also known.

GFRA1, or a fragment comprising its partial peptide, can be used as immunogens to obtain the antibodies of the present invention. The GFRA1 of the present invention can be derived from any species, preferably from a mammal such as a human, mouse, or rat, and more preferably from a human. The human GFRA1 nucleotide sequence and amino acid sequence are known (NM_005264). The cDNAnucleotide sequence of GFRA1 is described in SEQ ID NO: 1, and the amino acid sequences coded by that nucleotide sequence is described in SEQ ID NO:2. One skilled in the art can routinely isolate genes comprising the provided nucleotide sequence, preparing a fragment of the sequence as required, and obtain a protein comprising the target amino acid sequence.

For example, the gene coding the GFRA1 protein or its fragment can be inserted into a known expression vector, and used to transform host cells. The desired protein, or its fragment, can be collected from inside or outside host cells using arbitrary and standard methods, and can also be used as an antigen. In addition, proteins, their lysates, and chemically-synthesized proteins can be used as antigens. Furthermore, cells expressing the GFRA1 protein or a fragment thereof can themselves be used as immunogens.

When using a peptide fragment as the GFRA1 immunogen, it is particularly preferable to select an amino acid sequence which comprises a region predicted to be an extra-cellular domain. The existence of a signal peptide is predicted from positions 1 to 19 on the N-terminal of GFRA1 (Jing S. et al., Cell. (1996) Jun 28;85 (7):1113-24.). Thus, for example, a region other than the N-terminal signal peptide (19 amino acid residues) is preferred as the immunogen for obtaining the antibodies of the present invention. That is to say, antibodies that bind to GFRA1 extra-cellular domains are preferred as the antibodies of the present invention.

Therefore, preferable antibodies in the present invention are antibodies equipped with an Fc essential to effector function, and a variable region that can bind to an extra-cellular GFRA1 domain. When aiming for administration to humans, it is preferable to be equipped with an IgG Fc.

Any mammal can be immunized with such an antigen. However, it is preferable to consider compatibility with parent cells used in cell fusion. Generally, rodents, lagomorphs, or primates are used.

Rodents include, for example, mice, rats, and hamsters. Lagomorphs include, for example, rabbits. Primates include, for example, catarrhine (old world) monkeys such as *Macaca fascicularis, Macaca mulatta,* Sacred baboons, and chimpanzees.

Methods for immunizing animals with antigens are well known in the field. Intraperitoneal or subcutaneous antigen injections are standard methods for immunizing mammals. Specifically, antigens can be diluted and suspended in an appropriate amount of phosphate buffered saline (PBS), physiological saline, or so on. As desired, antigen suspensions can be mixed with an appropriate amount of a standard adjuvant such as Freund's complete adjuvant, and administered to mammals after emulsification. Subsequently, it is preferable that antigens mixed with an appropriate amount of Freund's incomplete adjuvant are administered in multiple doses every four to 21 days. An appropriate carrier can also be used for immunization. After carrying out immunization as outlined above, standard methods can be used to examine serum for an increase in the desired antibody level.

Polyclonal antibodies against the GFRA1 protein can be prepared from immunized mammals whose serum has been investigated for an increase in the desired antibodies. This can be achieved by collecting blood from these animals, or by using an arbitrary, usual method to isolate serum from their blood. Polyclonal antibodies comprise serum that comprises polyclonal antibodies, and fractions that comprise polyclonal antibodies which can be isolated from serum. IgG and IgM can be prepared from fractions that recognize GFRA1 protein by using, for example, an affinity column coupled to GFRA1 protein, and then further purifying this fraction using protein A or protein G columns. In the present invention, antiserum can be used as is as polyclonal antibodies. Alternatively, purified IgG, IgM, or such can also be used.

To prepare monoclonal antibodies, immunocytes are collected from mammals immunized with antigens, investigated for the increase of the desired antibody level in serum (as above), and applied in cell fusion. Immunocytes for use in cell fusion preferably come from the spleen. Other preferred parent cells for fusion with the above immunogens include, for example, mammalian myeloma cells, and more preferably, myeloma cells that have acquired properties for selection of fusion cells by pharmaceutical agents.

The above immunocytes and myeloma cells can be fused using known methods, for example the methods of Milstein *et al.* (Galfre, G. and Milstein, C., Methods. Enzymol, 1981, 73. 3-46).

Hybridomas produced by cell fusion can be selected by culturing in a standard selective medium such as HAT medium (medium comprising hypoxanthine, aminopterin, and thymidine). Cell culture in HAT medium is usually continued for several days to several weeks, a period sufficient enough to kill all cells other than the desired hybridomas (unfused cells). Standard limiting dilutions are then carried out, and hybridoma cells that produce the desired antibodies are screened and cloned.

Non-human animals can be immunized with antigens for preparing hybridomas in the above method. In addition, human lymphocytes from cells infected with EB virus or such, can be immunized *in vitro* using proteins, cells expressing proteins, or suspensions of the same. The immunized lymphocytes are then fused with human-derived myeloma cells able to divide unlimitedly (U266 and so on), thus obtaining hybridomas that produce the desired human antibodies which can bind the protein (Unexamined Published Japanese Patent Application No. (JP-A) Sho 63-17688).

The obtained hybridomas are then transplanted to mice abdominal cavities, and ascites are extracted. The obtained monoclonal antibodies can be purified using, for example, ammonium sulfate precipitation, protein A or protein G columns, DEAE ion exchange chromatography, or affinity columns coupled to the proteins of the present invention. The antibodies of the present invention can be used not only in purifying and detecting the proteins of the present invention, but also as candidates for agonists and antagonists of the proteins of the present invention. These antibodies can also be applied to antibody therapies for diseases related to the proteins of the present invention. When the obtained antibodies are administered to human bodies (antibody therapy), human antibodies or humanized antibodies are preferred due to their low immunogenicity.

For example, transgenic animals comprising a repertoire of human antibody genes can be immunized with antigens selected from proteins, protein-expressing cells, or suspensions of the same. Antibody-producing cells are then recovered from the animals, fused with myeloma cells to yield hybridomas, and anti-protein human antibodies can be prepared from these hybridomas (see International Publication No. 92-03918, 93-2227, 94-02602, 94-25585, 96-33735, and 96-34096).

Alternatively, immunocytes such as immunized lymphocytes that produce antibodies, can be immortalized using cancer genes, and used to prepare monoclonal antibodies.

Monoclonal antibodies obtained in this way can be prepared using methods of genetic engineering (for example, see Borrebaeck, C.A.K. and Larrick, J.W., Therapeutic Monoclonal Antibodies, MacMillan Publishers, UK, 1990). For example, recombinant antibodies can be prepared by cloning DNAs that encode antibodies from immunocytes such as hybridomas or immunized lymphocytes that produce antibodies; then inserting these DNAs into appropriate vectors; and transforming these into host cells. Recombinant antibodies prepared as above can also be used in the present invention.

The antibodies can be modified by binding with a variety of molecules such as polyethylene glycols (PEGs). Antibodies modified in this way can also be used in the present invention. Modified antibodies can be obtained by chemically modifying antibodies. These kinds of modification methods are conventional to those skilled in the art. The antibodies can also be modified by other proteins. Antibodies modified by protein molecules can be produced using genetic engineering. That is, target proteins can be expressed by fusing antibody genes with genes that code for modification proteins. For example, antibody effector function may be enhanced on binding with cytokines or chemokines. In fact, the enhancement of antibody effector function for proteins fused with IL-2, GM-CSF, and such has been confirmed (Human Antibody, 10: 43-49,2000). IL-2, IL-12, GM-CSF, TNF, eosinophil chemotactic substance (RANTES) and so on can be included in cytokines or chemokines that enhance effector function.

Alternatively, antibodies of the present invention can be obtained as chimeric antibodies which comprise a non-human antibody-derived variable region and a human antibody-derived constant region, or as humanized antibodies which comprise a non-human antibody-derived complementarity determining region (CDR), a human antibody-derived framework region (FR), and a constant region. Such antibodies can be produced using known methods.

Antibodies obtained as above can be purified until uniform. For example, antibodies can be purified or separated according to general methods used for purifying and separating proteins. For example, antibodies can be separated and isolated using appropriately selected combinations of column chromatography, comprising but not limited to affinity chromatography, filtration, ultrafiltration, salt precipitation, dialysis, SDS polyacrylamide gel electrophoresis, isoelectric focusing, and so on (Antibodies : A Laboratory Manual, Harlow and David, Lane (edit.), Cold Spring Harbor Laboratory, 1988).

Protein A columns and Protein G columns can be used as affinity columns. Exemplary protein A columns in use include Hyper D, POROS, and Sepharose F.F (Pharmacia).

Exemplary chromatography (excluding affinity chromatography) include ion exchange chromatography, hydrophobic chromatography, gel filtration, reverse phase chromatography, and adsorption chromatography ("Strategies for Protein Purification and Characterization: A Laboratory Course Manual". Daniel R. Marshak et al., Cold Spring Harbor Laboratory Press, 1996). The chromatography can be performed according to the procedure of liquid phase chromatographies such as HPLC or FPLC.

For example, the antigen-binding activity of the antibodies of the present invention can be measured by using absorbance measurements, enzyme linked immunosorbent assays (ELISA), enzyme immunoassays (EIA), radioimmunoassays (RIA) and/or immunofluorescence methods. In ELISA, an antibody of the present invention is immobilized on a plate, a protein of the present invention is added to the plate, and then a sample comprising the desired antibody such as the culture supernatant of cells that produce the antibody or purified antibody is added. A secondary antibody that recognizes the primary antibody and has been tagged with an enzyme such as alkaline phosphatase is then added, and the plate is incubated. After washing, an enzyme substrate such as p-nitrophenyl phosphate is added to the plate, absorbance is measured, and the antigen-binding activity of the samples is evaluated. Protein fragments (C-terminal or N-terminal fragments, and such) can be used in the same way as proteins. The binding activity of the antibodies of the present invention can be evaluated using BIAcore (Pharmacia).

In addition, by following the methods outlined in the Examples, antibody effector function can also be evaluated. For example, target GFRA1-expressing cells are incubated with effector cells in the presence of an antibody whose effector function is to be evaluated. If target cell destruction is detected, the antibody can be confirmed to comprise effector function that induces ADCC. The level of observed target cell destruction, in the absence of either antibodies or effector cells, can be compared as a control with the level of effector function. Cells which clearly express GFRA1 can be used as the target cells. Specifically, a variety of cell lines confirmed to express GFRA1 in the Examples can be used. These cell lines can be obtained from cell banks. In addition, monoclonal antibodies which comprise more powerful effector function can be selected.

In the present invention, anti-GFRA1 antibodies can be administered to humans or other animals as pharmaceutical agents. In the present invention, animals other than humans to which the antibodies can be administered include mice, rats, guinea pigs, rabbits, chickens, cats, dogs, sheep, pigs, cows, monkeys, baboons, and chimpanzees. The antibodies can be directly administered to subjects, and in addition, can be formulated into dosage forms using known pharmaceutical formulation methods. For example, depending on requirements, they can be parenterally administered in an injectable form such as a sterile solution or suspension with water or other arbitrary pharmaceutically acceptable fluid. For example, this kind of compounds can be mixed with acceptable carriers or solvents, specifically sterile water, physiological saline, vegetable oils, emulsifiers, suspension agents, surfactants, stabilizers, flavoring agents, excipients, solvents, preservatives, binding agents and the like, into a generally accepted unit dosage essential for use as a pharmaceutical agent.

Other isotonic solutions comprising physiological saline, glucose, and adjuvants (such as D-sorbitol, D-mannose, D-mannitol, and sodium chloride) can be used as the injectable aqueous solution. They can also be used with appropriate solubilizers such as alcohols, specifically ethanols and polyalcohols (for example, propylene glycols and polyethylene glycol), and non-ionic surfactants (for example polysorbate 80^{™} or HCO-50).

Sesame oils or soybean oils can be used as an oleaginous solution, and benzyl benzoate or benzyl alcohols can be used with them as a solubilizer. Buffer solutions (phosphate buffers, sodium acetate buffers, or so on), analgesics (procaine hydrochloride or such), stabilizers (benzyl alcohol, phenols, or so on), and antioxidants can be used in the formulation. The prepared injections can be packaged into appropriate ampules.

In the present invention, the anti-GFRA1 antibodies can be administered to patients, for example, intraarterially, intravenously, or percutaneously, or intranasally, transbronchially, locally, or intramuscularly. Intravascular (intravenous) administration by drip or injection is an example of a general method for systematic administration of antibodies to breast, gastric, liver, renal or lung cancer patients. Methods of locally concentrating antibody agents to the primary focus or metastatic focus in the lung include local injection using a bronchoscope (bronchoscopy) and local injection under CT guidance or with thoracoscopy. Methods of locally concentrating antibody agents to the primary focus or metastatic focus in the liver include local injection using a hepatic portal injection or arterial infusion. In addition, methods in which an intraarterial catheter is inserted near a vein that supplies nutrients to cancer cells to locally inject anti-cancer agents such as antibody agents, are effective as local control therapies for metastatic focuses as well as primary focuses of breast, gastric, liver, renal or lung cancer.

Although dosage and administration methods vary according to patient body weight and age, and administration method, these can be routinely selected by one skilled in the art.

In addition, DNA encoding an antibody can be inserted into a vector for gene therapy, and the vector can be administered for therapy. Dosage and administration methods vary according to patient body weight, age, and condition, however, one skilled in the art can select these appropriately.

Anti-GFRA1 antibodies can be administered to living bodies in an amount such that cytotoxicity based on effector function against GFRA1-expressing cells can be confirmed. For example, although there is a certain amount of difference depending on symptoms, anti-GFRA1 antibody dosage is 0.1 mg to 250 mg/kg per day. Usually, the dosage for an adult (of weight 60 kg) is 5 mg to 17.5 g/day, preferably 5 mg to 10 g/day, and more preferably 100 mg to 3 g/day. The dosage schedule is from one to ten times over a two to ten day interval, and for example, progress is observed after a three to six times administration.

Although the antibodies of the invention retain effector function, in some embodiments, cytotoxic agents can be linked to the antibodies using well known techniques. Cytotoxic agents are numerous and varied and include, but are not limited to, cytotoxic drugs or toxins or active fragments of such toxins. Suitable toxins and their corresponding fragments include diphtheria A chain, exotoxin A chain, ricin A chain, abrin A chain, curcin, crotin, phenomycin, enomycin, auristatin and the like. Cytotoxic agents also include radiochemicals made by conjugating radioisotopes to the antibodies of the invention or binding of a radionuclide to a chelating agent that has been covalently attached to the antibody. Methods for preparing such conjugates are well known in the art.

In addition, the present invention provides immunogenic compositions for inducing antibodies comprising effector functions against GFRA1-expressing cells, where the compositions comprise as an active ingredient GFRA1 or an immunologically active GFRA1 fragment, or a DNA or cell which can express the same. Alternatively, the present invention relates to uses of GFRA1 or an immunologically active GFRA1 fragment, or a DNA or cell which can express the same in the production of immunogenic compositions for inducing antibodies comprising effector functions against GFRA1-expressing cells..

The administration of anti-GFRA1 antibodies damages cancer cells by the effector function of those antibodies. Thus, if GFRA1 antibodies can be induced *in vivo,* therapeutic effects equivalent to the antibody administration can be achieved. When administering immunogenic compositions comprising antigens, target antibodies can be induced *in vivo.* The immunogenic compositions of the present invention thus are particularly useful in vaccine therapy against GFRA1-expressing cells. Thus, the immunogenic compositions of the present invention are effective as, for example, vaccine compositions for breast, gastric, liver, renal or lung cancer therapies.

The immunogenic compositions of the present invention can comprise GFRA1 or an immunologically active GFRA1 fragment, as an active ingredient. An immunologically active GFRA1 fragment refers to a fragment that can induce anti-GFRA1 antibodies which recognize GFRA1 and comprise effector function. Below, GFRA1 and the immunologically active GFRA1 fragment are described as immunogenic proteins. Whether a given fragment induces target antibodies can be determined by actually immunizing an animal, and confirming the activity of the induced antibodies. Antibody induction and the confirmation of its activity can be carried out, for example, using methods described in Examples. For example, fragments comprising an amino acid sequence corresponding to GFRA1 position 24 to 465 can be used as the immunogens of the present invention.

The immunogenic compositions of the present invention comprise pharmaceutically acceptable carriers as well as immunogenic proteins, the active ingredients. If necessary, the compositions can also be combined with an adjuvant. Killed tuberculosis bacteria, diphtheria toxoid, saponin and so on can be used as the adjuvant.

Alternatively, DNAs coding for the immunogenic proteins, or cells retaining those DNAs in an expressible state, can be used as the immunogenic compositions. Methods for using DNAs expressing the target antigen as immunogens, so-called DNA vaccines, are well known. DNA vaccines can be obtained by inserting a DNA encoding GFRA1 or its fragment into an appropriate expression vector.

Retrovirus vectors, adenovirus vectors, adeno-associated virus vectors, Sendai virus vectors or such can be used as the vector. In addition, DNAs in which a DNA encoding an immunogenic protein is functionally connected downstream of a promoter can be directly introduced into cells as naked DNA, and then expressed. Naked DNA can be encapsulated in ribosomes or viral envelope vectors and introduced into cells.

The GFRA1 polypeptides and polynucleotides of the invention can also be used for the induction of an immune *response in vivo,* including production of antibodies and cytotoxic T lymphocytes (CTL) specific for GFRA1 expressing cells. In such methods, CTL induction by a desired peptide can be achieved by presenting the peptide to a T cell via an antigen presenting cell (APC) either *in vivo* or *ex vivo.*

For example, patient blood cells e.g., peripheral blood mononuclear cells (PBMC) are collected, transformed using a vector that can express the immunogenic proteins, and returned to the patient. Transformed bloods cells produce the immunogenic proteins inside the body of the patient, and induce the target antibodies. Alternatively, PBMCs of the patient are collected, the cells are contacted with the polypeptide *ex vivo,* and following the induction of APCs or CTLs, the cells may be administered to the subject. APCs or CTLs induced *in vitro* can be cloned prior to administration. By cloning and growing cells having high activity of damaging target cells, cellular immunotherapy can be performed more effectively. Furthermore, APCs and CTLs isolated in this manner may be used for cellular immunotherapy not only against individuals from whom the cells are derived, but also against similar types of tumors from other individuals.

Generally, when using a polypeptide for cellular immunotherapy, efficiency of the CTL-induction is known to be increased by combining a plurality of polypeptides having different structures and contacting them with APCs, particularly, dendritic cells. Therefore, when stimulating APCs with protein fragments, it is advantageous to use a mixture of multiple types of fragments.

The induction of anti-tumor immunity by a polypeptide can be confirmed by observing the induction of antibody production against tumors. For example, when antibodies against a polypeptide are induced in a laboratory animal immunized with the polypeptide, and when growth of tumor cells is suppressed by those antibodies, the polypeptide is deemed to have the ability to induce anti-tumor immunity.

When DNAs encoding the immunogenic proteins, or cells transformed with the same are used as immunogenic compositions of the present invention, they can be combined with immunogenic proteins as well as carrier proteins that enhance their immunogenic properties.

As noted above, the present invention provides methods for inducing antibodies which comprise effector function against GFRA1-expressing cells, where the methods comprise the step of administering GFRA1, an immunologically active GFRA1 fragment, or DNA or cells that can express the same. The methods of the present invention induce antibodies that comprise effector function that damages GFRA1-expressing cells such as breast, gastric, liver, renal or lung cancers. As a result, therapeutic effects for breast, gastric, liver, renal or lung cancers and so on can be obtained.

Each day, 0.1 mg to 250 mg per kilogram of the immunogenic compositions of the present invention can be administered orally or parenterally. Parenteral administration includes subcutaneous injection and intravenous injection. The administrative dose for a single adult is usually mg to 17.5 g/day, preferably 5 mg to 109/day, and more preferably 100 mg to 3 g/day.

### Brief Description of the Drawings

- Figure. 1: are photographs depicting the result of Semiquantitative RT-PCR analysis for the GFRA1 gene in cancer cells. A; for breast cancer cell lines. B; for gastric cancer cell lines. C, for liver cancer cell lines. D, for renal cancer cell lines. E, for lung cancer cell lines. The expression level of harceptin target gene c-erbB2 gene for breast cancer is indicated in panel A (positive control).
- Figure 2: shows the results of an ADCC assay using Herceptin against (A) MDA-MB-453 over-expressed *c-erbB-2* gene and (B) MCF-7 low-expressed *c-erbB-2* gene.
- Figure: 3 shows the results of an ADCC assay using anti-GFRA1 antibody Br003 against GFRA1-over- and low-expressing breast cancer cell line, (A) MCF-7 and (B) MDA-MB-453, respectively.
- Figure 4: shows the results of an ADCC assay using anti-GFRA1 antibody Br003 against GFRA1-over-eapressing gastric cancer cell line, MKN1.
- Figure 5: shows the results of an ADCC assay using anti-GFRA1 antibody Br003 against GFRA1-over-expressing liver cancer cell line, SNU-398.
- Figure 6: shows the results of an ADCC assay using anti-GFRA1 antibody Br003 against GFRA1-over-expressing renal cancer cell line, ACHN.
- Figure 7: shows the results of an ADCC assay using anti-GFRA1 antibody Br003 against GFRA1-over-expressing lung cancer cell line, NCI-H1793.

### Best Mode for Carrying Out the Invention

Below, the present invention is further explained based on Examples.

### Cell line:

Human breast, gastric, liver, renal or lung cancer cell lines were propagated as a monolayer in an appropriate medium with 10% fetal bovine serum. The cell lines used in the experiment are shown in Table 1.

**Table 1**

| **Breast cancer Cell line** | | |
|---|---|---|
| Cell line | Medium | Place obtained |
| BT-20 | E-MEM^{*1} +10% FBS | American Type Culture Collection (ATCC); HTB-19 |
| BT-474 | D-MEM^{*2} +10% FBS | ATCC; HTB-20 |
| BT-549 | RPMI+10% FBS | ATCC; HTB-122 |
| HCC1143 | RPMI+10% FBS | ATCC; CRL-2321 |
| HCC1395 | RPMI+10% FBS+2mM L-glutamin | ATCC; CRL-2324 |
| HCC1500 | RPMI+10% FBS+2mM L-glutamin | ATCC;CRL.2329 ATCC; CRL-2329 |
| HCC1937 | RPMI+10% FBS+2mM L-glutamin | ATCC;CRL.2336 ATCC; CRL-2336 |
| MCF-7 | E-MEM^{*1} +10% FBS | ATCC; HTB-22 |
| MDA-MB-157 | L15^{*3} +10% FBS | ATCC; HTB-24 |
| MDA-MB-231 | L15^{*3} +10% FBS | ATCC; HTB-26 |
| MDA-MB-4355 | L15^{*3} +10% FBS | ATCC; HTB-129 |
| MDA-MB-453 | McCoy^{*4}+10% FBS | ATCC; HTB-131 |
| SK-BR-3 | RPMI+10% FBS | ATCC; HTB-30 |
| T-47D | RPMI+10% FBS+2mM L-glutamin | ATCC; HTB-133 |
| ZR-75-1 | E-MEM^{*1} +10% FBS | ATCC; CRL-1500 |

| **Gastric cancer Cell line** | | |
|---|---|---|
| Cell line | Medium | Place obtained |
| MKN1 | RPMI+10% FBS | Health Science Research Resources Bank (HSRRB); JCRB0252 |
| MKN7 | RPMI+10% FBS | HSRRB; JCRB1025 |
| MKN45 | RPMI+10% FBS | HSRRB; JCRB0254 |
| MKN74 | RPMI+10% FBS | HSRRB; JCRB0255 |

| **Liver cancer Cell line** | | |
|---|---|---|
| Cell line | Medium | Place obtained |
| Alexander | D-MEM^{*2} +10% FBS | HSRRB; IFO50069 |
| Hep G2 | D-MEM^{*2} +10% FBS | HSRRB; JCRB1054 |
| HUH-6 Clone 5 | E-MEM^{*1} +10% FBS | HSRRB; JCRB0401 |
| HuH-7 | D-MEM^{*2} +10% FBS | HSRRB; JCRB0403 |
| SNU-398 | RPMI+10% FBS (heat inactivated) | ATCC; CRL-2233 |
| SNU-423 | RPMI+10% FBS | ATCC; CRL-2238 |
| SNU-449 | RPMI+10% FBS | ATCC; CRL-2234 |
| SNU-475 | RPMI+10% FBS | ATCC; CRL-2236 |

| **Renal cancer Cell line** | | |
|---|---|---|
| Cell line | Medium | Place obtained |
| ACHN | RPMI+5% FBS | ATCC; CRL-1611 |
| 786-O | RPMI+10% FBS | ATCC; CRL-1932 |
| A-498 | E-MEM^{*1} +10% FBS | ATCC; HTB-44 |
| Caki-1 | McCoy^{*4}+10% FBS | HSRRB; JCRB0801 |
| Caki-2 | McCoy^{*4}±10% FBS | ATCC; HTB-47 |

| **Lung cancer Cell line** | | |
|---|---|---|
| Cell line | Medium | Place obtained |
| NCI-H23 | RPMI+10% FBS | ATCC; CRL-5800 |
| NCI-H358 | RPMI+10% FBS | ATCC; CRL-5807 |
| NCI-H596 | RPMI+10% FBS | ATCC; HTB-178 |
| NCI-H1650 | RPMI+10% FBS | ATCC; CRL-5883 |
| NCI-H1793 | F12^{*5}+ D-MEM^{*2}+10% FBS | ATCC; CRL-5896 |
| PC-14 | RPMI+10% FBS | RIKEN Bioresource Center |
| SK-MES-1 | E-MEM^{*1} +10% FBS + 2mM L₋glutamin | ATCC; HTB-58 |
| SK-LU-1 | E-MEM^{*1} +10% FBS + 2mM L₋glutamin | ATCC ; HTB-57 ATCC ; HTB-57 |
| SW900 | L15^{*3} +10% FBS | ATCC; HTB-59 |
| SW1573 | L15^{*3} +10% FBS | ATCC; CRL-2170 |
| A549 | RPMI+10% FBS | ATCC; CCL-185 |
| NCI-H522 | RPMI+10% FBS | ATCC; CRL-5810 |
| PC-3 | E-MEM^{*1}+10% FBS | HSRRB; JCRB0077 |

| | | |
|---|---|---|
| ^{*1} Eagle's Minimal Essential medium ^{*2} Dulbecco's Modified Eagle's medium ^{*3} Leibovitz's L-15 medium ^{*4} McCoy's 5A medium Modified ^{*5} F-12 Nutrient Mixture (HAM) | | |

Furthermore, the following cell lines were used in ADCC assays using anti-GFRA1 antibodies: Breast adenocarcinoma (BC): MCF-7
Breast carcinoma: MDA-MB-453.
Stomach adenosquamous carcinoma: MKN1.
Hepatocellular carcinoma (HCC): SNU-398.
Renal cell adenocarcinoma (RCC): ACHN.
Non-small cell lung carcinoma (NSCLC): NCI-H1793.

### Antibody production:

According to standard protocols, individual protein specific antibodies were produced using His-tagged fusion proteins expressed in bacteria as immunogens. These fusion proteins comprised a protein portion that corresponded to one part of the protein (residues 24 to 465).

### Semiquantitative RT-PCR for GFRA1 and c-erbB2:

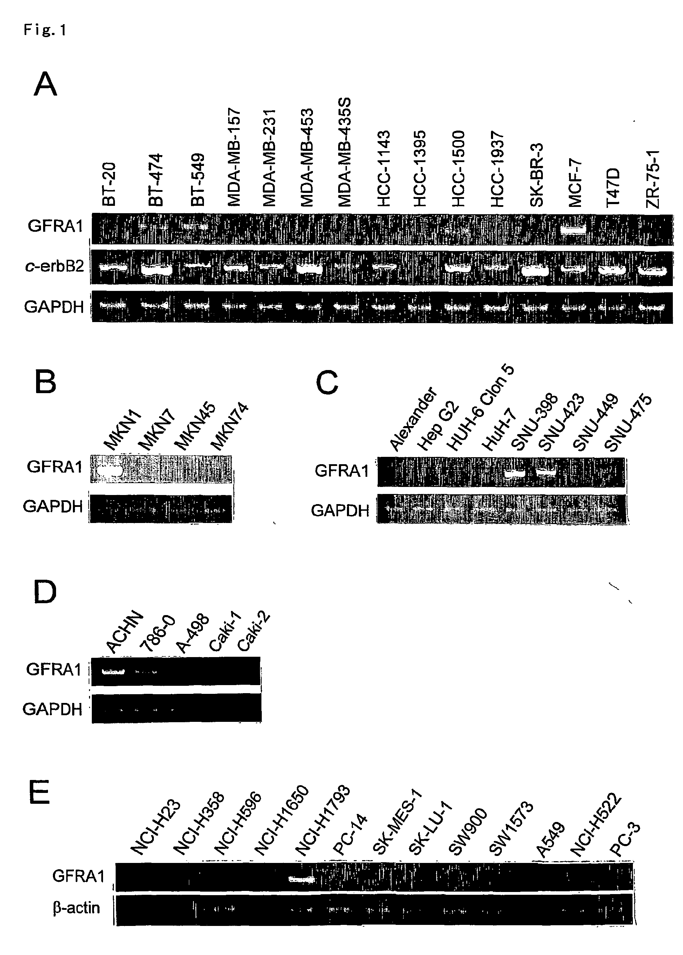

Total RNA was extracted from the cell lines using the Rneasy® Kit (QIAGEN). In addition, mRNA was purified from total RNA by Oligo (dT)-cellulose column (Amersham Biosciences) and synthesized to first-strand cDNA by reverse transcription (RT) using the SuperScript First-Strand Synthesis System (Invitrogen). It was prepared appropriate dilutions of each first-stranded cDNA for subsequent PCR amplification by monitoring GAPDH as a quantitative control. The primer sequences the present inventors used were
5'-CTGAAGCAGAAGTCGCTCTA-3' (SEQ.ID.NO.3) and
5'-GACAGCTGCTGACAGACCTT-3' (SEQ.ID.NO.4) for GFRA1,
5'-GTCAGTGGTGGACCTGACCT-3' (SEQ.ID.NO.5) and
5'-GGTTGAGCACAGGGTACTTTATT 3' (SEQ.ID.NO.6) for GAPDH. All PCR reactions involved initial denaturation at 94°C for 2 min and consisted of 94°C for 30 s, 58°C for 30 s, and 72°C for 1 min by 21 cycles (for GAPDH) or 30-40 cycles (for GFRA1), on a GeneAmp PCR system 9700 (PE Applied Biosystems).
The over-expression of GFRA1 was found in breast cancer cell line MCF-7 (Fig. 1). In addition, to elucidate the efficacy of anti-GFRA1 antibody (Br003) on various cancers, the expression of GFRA1 was confirmed. The over-expression of GFRA1 was decided in gastric cancer cell line MKN1, liver cancer cell line SNU-398, renal cancer cell line ACHN, and lung cancer cell line NCI-H1793.

### Flow cytometry analysis:

Cancer cells (5x 10⁶) were incubated at 4°C for 30 minuets with the purified polyclonal antibodies (pAb) or rabbit IgG (the control). Cells were washed with phosphate buffer solution (PBS) and then incubated at 4°C for 30 minutes in FITC-labeled Alexa Fluor 488. The cells were again washed in PBS, and analyzed on a flow cytometer (FACSCalibur^{®}, Becton Dickinson) and then analyzed by BD CellQuest^{™} Pro software (Becton Dickinson). Mean fluorescence intensity (MFI) was defined as a ratio of the flow cytometric intensity (intensity by each protein specific antibody/intensity by rabbit IgG).

Using anti-GFRA1 antibodies Br003, GFRA1 expression was investigated for MCF-7, MKN1, SNU-398, ACHN, and NCI-H1793 cells. As a result, a higher proportion of anti-GFRA1 antibodies (Br003) bound to MCF-7, MKN1, SNU-398, ACHN, and NCI-H1793 cells (MFI(Mean fluorescence intensity): 155.4, 5.2, 9.3, 78.5, and 9.4, respectively) than did rabbit IgG (the control).

### ADCC assays:

Target cells were exposed with 0.8µM of calcein acetoxymethyl estel (Calcein-AM, DOJINDO) at 37°C for 30 minutes. Calcein-AM becomes fluorescent after the cleavage of calcein-AM by cellular esterases that produce a fluorescent derivate calcein. Target cancer cells were washed two times before being added to the assay, and cells were then plated on 96-well U-bottom plates (4x 10³ cells/well). Human peripheral blood mononuclear cells (PMBC) were harvested from a healthy person, separated using Ficoll-Paque (Amersham Biosciences) density gradient centrifugation, and then used as effector cells. Target cancer cells (T) and effector cells (E) were co-incubated in 200 µl of AIM-V medium in 96-well U-bottom plates at various E:T ratios (50:1, 25:1, 12.5:1, and 6.25:1) with Br003 anti-GFRA1 antibody (2 µg/well) or control antibody Herceptin (2µg/well, Roche). This incubation was carried out in triplicate, in 200 µL of AIM-V medium (Life Technologies, Inc), at 37°C for six hours. Control assays included the incubation of target cells with only anti-GFRA1 antibody Br003 or effector cells. Herceptin was used as a control in some experiments.

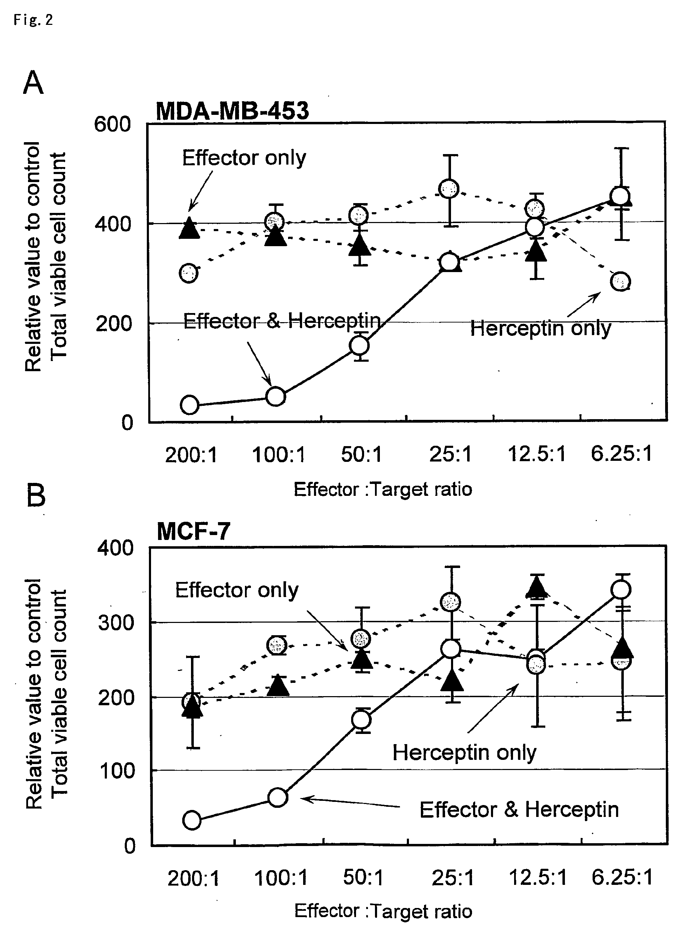

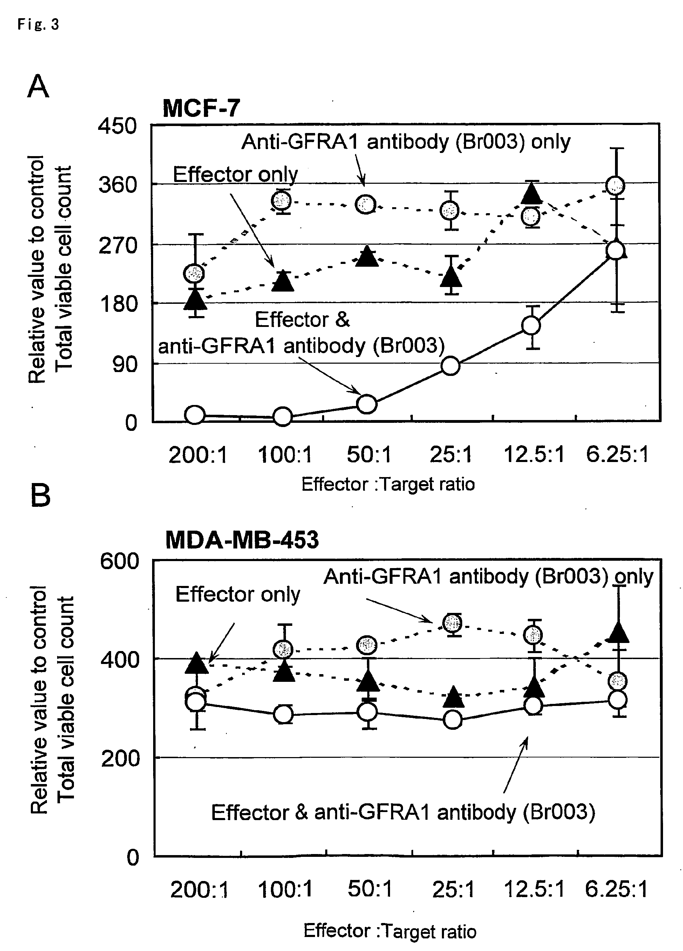

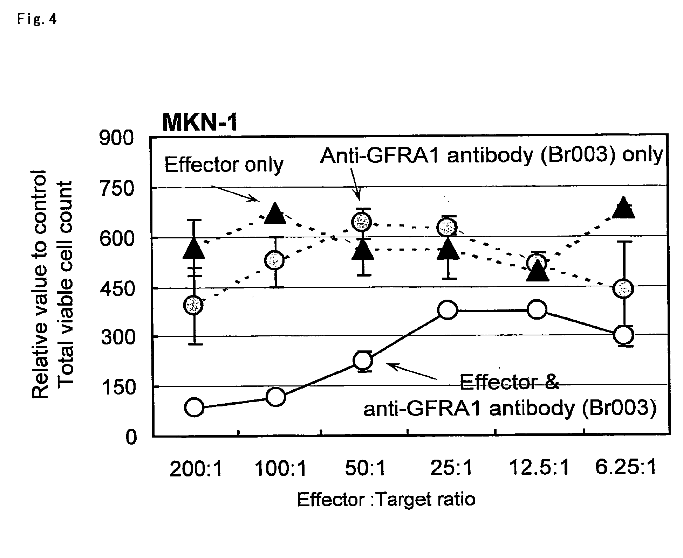

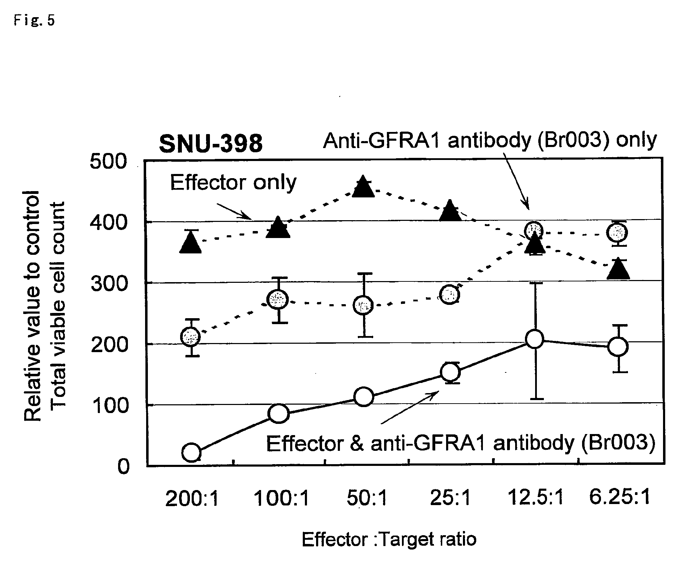

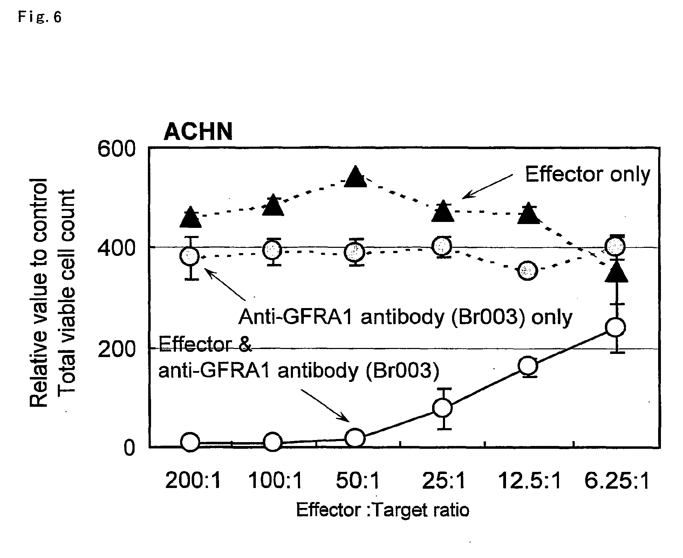

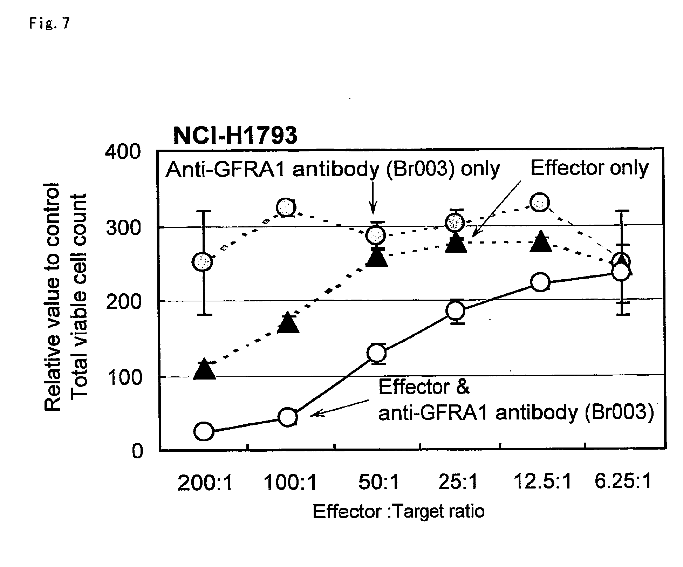

The ADCC effects of anti-GFRA1 antibody (Br003) for MCF-7, MKN1, SNU-398, ACHN, and NCI-H1793 cells were evaluated based on the fluorescent images of viable cells were rapidly acquired using the IN Cell Analyzer 1000 (Amersham Bioscience). These images were numerically converted as viable cell count by counting the fluorescent object or vesicle using Developer tool ver.5.21 software (Amersham Bioscience).
Control assays were carried out by incubating target cells with only Br003 anti-GFRA1 antibody or only effector cells. Herceptin was used as a control in several experiments (Fig.2A,2B). Direct cell damage of MCF-7, MKN1, SNU-398, ACHN, and NCI-H1793 cells by Br003 anti-GFRA1 antibody itself was not observed. However, Br003 induced ADCC in MCF-7, MKN1, SNU-398, ACHN, and NCI-H1793 cells that over-expressed GFRA1 (Fig.3A,4-7),
while no effect against MDA-MB-453 cells with GFRA1 low-expression (Fig.3B).

### Industrial Applicability

The present invention is based, at least in part, on the discovery that GFRA1-expressing cells can be damaged by antibody cytotoxicity. GFRA1 was identified by the present inventors as a gene strongly expressed in breast, gastric, liver, renal or lung cancers. Thus, treatment of disease associated with GFRA1-expressing cells, for example, breast, gastric, liver, renal or lung cancer is conveniently carried out using antibodies that bind to GFRA1. Results actually confirmed by the present inventors show cytotoxicity due to the effect of ADCC in breast, gastric, liver, renal or lung cancer cell lines, in the presence of GFRA1 antibodies

## Claims

1. A composition for use in treating GFRA1-expresssing cancer, the composition comprising an anti-GFRA1 antibody as the active ingredient, wherein the antibody comprises antibody effector function, wherein said effector function is either antibody-dependent cytotoxicity or complement-dependent cytotoxicity, or both.

2. Use of an anti-GFRA1 antibody for the preparation of a pharmaceutical composition for treating GFRA1-expresssing cancer, wherein said antibody comprises antibody effector function, wherein said effector function is either antibody-dependent cytotoxicity or complement-dependent cytotoxicity, or both.

3. The composition for use in treating GFRA1-expressing cancer of claim 1, or the use of claim 2, wherein the cancer is breast cancer, gastric cancer, liver cancer, renal cancer or lung cancer.

4. The composition for use in treating GFRA1-expressing cancer of claim 1 or 3, or the use of claim 2 or 3, wherein the anti-GFRA1 antibody is a monoclonal antibody.

## Patentansprüche

1. Zusammensetzung zur Verwendung in der Behandlung von GFRA-1 exprimierendem Krebs, umfassend einen Anti-GFRA-1-Antikörper als Wirkstoff, wobei der Antikörper Antikörpereffektorfunktion umfasst, wobei die Effektorfunktion entweder Antikörper-abhängige Cytotoxizität, Komplement-abhängige Cytotoxizität oder beides ist.

2. Verwendung eines Anti-GFRA-1-Antikörpers für die Herstellung eines Medikaments zur Behandlung von GFRA-1 exprimierendem Krebs, wobei der Antikörper Antikörpereffektorfunktion umfasst, wobei die Effektorfunktion entweder Antikörper-abhängige Cytotoxizität, Komplement-abhängige Cytotoxizität oder beides ist.

3. Zusammensetzung zur Verwendung in der Behandlung von GFRA-1 exprimierendem Krebs nach Anspruch 1 oder Verwendung nach Anspruch 2, wobei der Krebs Brustkrebs, Magenkrebs, Leberkrebs, Nierenkrebs oder Lungenkrebs ist.

4. Zusammensetzung zur Verwendung in der Behandlung von GFRA-1 exprimierendem Krebs nach Anspruch 1 oder 3 oder Verwendung nach Anspruch 2 oder 3, wobei der Anti-GFRA-1-Antikörper ein monoclonaler Antikörper ist.

## Revendications

1. Composition pour son utilisation dans le traitement d'un cancer exprimant GFRA1, la composition comprenant un anticorps anti-GFRA1 en tant qu'ingrédient actif, l'anticorps comprenant une fonction effectrice d'anticorps, ladite fonction effectrice étant soit une cytotoxicité anticorps-dépendante, soit une cytotoxicité complément-dépendante, soit les deux.

2. Utilisation d'un anticorps anti-GFRA1 pour la préparation d'une composition pharmaceutique pour traiter un cancer exprimant GFRA1, ledit anticorps comprenant une fonction effectrice d'anticorps, ladite fonction effectrice étant soit une cytotoxicité anticorps-dépendante, soit une cytotoxicité complément-dépendante, soit les deux.

3. Composition pour son utilisation dans le traitement d'un cancer exprimant GFRA1 selon la revendication 1, ou utilisation selon la revendication 2, dans laquelle le cancer est un cancer du sein, une cancer gastrique, une cancer du foie, une cancer rénal ou une cancer du poumon.

4. Composition pour son utilisation dans le traitement d'un cancer exprimant GFRA1 selon la revendication 1 ou 3, ou utilisation selon la revendication 2 ou 3, dans laquelle l'anticorps anti-GFRA1 est un anticorps monoclonal.
